(19) Europäisches Patentamt / European Patent Office / Office européen des brevets

(11) **EP 4 491 203 A1**

(12) **EUROPEAN PATENT APPLICATION**
published in accordance with Art. 153(4) EPC

(43) Date of publication:
**15.01.2025 Bulletin 2025/03**

(21) Application number: **22932588.1**

(22) Date of filing: **22.03.2022**

(51) International Patent Classification (IPC):
**A61L 29/08** (2006.01)  **A61M 25/10** (2013.01)
**A61L 31/00** (2006.01)

(52) Cooperative Patent Classification (CPC):
**A61L 29/08; A61L 31/00; A61M 25/10**

(86) International application number:
**PCT/CN2022/082303**

(87) International publication number:
**WO 2023/178519 (28.09.2023 Gazette 2023/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Lepu Medical Technology (Beijing) Co.,
Ltd.
Beijing 102200 (CN)**

(72) Inventors:
• **MA, Cuijie
Beijing 102200 (CN)**
• **ZHAO, Lixiao
Beijing 102200 (CN)**
• **LIU, Yangyang
Beijing 102200 (CN)**
• **HE, Zhihao
Beijing 102200 (CN)**
• **WANG, Zhe
Beijing 102200 (CN)**

(74) Representative: **Handley, Matthew Edward et al
Venner Shipley LLP
St James's
79 Oxford Street
Manchester M1 6EG (GB)**

(54) **DRUG COATING, DRUG BALLOON, PREPARATION METHOD FOR DRUG BALLOON AND USE THEREOF**

(57) A drug coating, a drug balloon, a preparation method for the drug balloon and use thereof. The drug coating for use in the drug balloon comprises a drug and a carrier, the carrier being a phenolic compound or a pyrone compound, and the mass ratio of the drug to the carrier being 1:(0.01-10). The drug balloon is obtained by coating the surface of a balloon with the drug coating. The phenolic compound or pyrone compound is used as a new carrier, so that the carrier selection efficiency of the drug coating is improved, an ideal crystal form can be obtained, the quality of the drug coating is remarkably improved, and the retention time of the drug in the treatment of blood vessels or other lumen stenosis diseases is prolonged, thereby enabling the drug balloon catheter to better inhibit restenosis of the lumen in the treatment of a narrow blood vessel, a narrow urethra, or a narrow ureter.

Figure 1

EP 4 491 203 A1

**Description**

**TECHNICAL FIELD**

The present application relates to the technical field of drug balloons, and specifically relates to a drug coating, a drug balloon and a preparation method for drug balloon and use thereof.

**BACKGROUND**

The drug balloon catheter is an interventional medical device consisting of a catheter and a drug balloon. The drug balloon consists of a balloon and a drug coating coated on its surface. The drug balloon catheter is mainly used to dilate the stenotic part of the blood vessel. While dilating the blood vessel, it releases the drug onto the tunica intima, thereby inhibiting restenosis of the blood vessel.

The formula of a drug coating generally comprises a drug and a carrier. The drug and the carrier are dissolved in a solvent and then coated on the surface of the balloon to obtain a drug balloon. At present, in domestic and international technologies, the carriers used to obtain drug balloons are generally contrast media, ester compounds, heparin, castor oil or hydrophilic dyes, sugars and sugar derivatives, polymers, etc., and the drug is generally taxol, rapamycin, etc. In short, there are many choices for drug coating formulas. However, for most of the drug coatings obtained from current formulas, it is difficult to control their crystallization state, and it is difficult to ensure the retention time of the drug when treating blood vessels or other luminal stenosis diseases.

**SUMMARY OF THE INVENTION**

Therefore, the technical problem to be solved by the present application is to overcome the shortcomings that it is difficult to control the crystallization state of most of drug coatings obtained from formulas in the prior art and to ensure the retention time of drug balloons when treating blood vessels or other luminal stenosis diseases.

To this end, the present application provides a drug coating, comprising a drug and a carrier, wherein the carrier is a phenolic compound or a pyrone compound, and a mass ratio of the drug to the carrier is 1: (0.01-10).

Further, the phenolic compound is at least one selected from the group consisting of eugenol, thymol, grape polyphenol, tea polyphenol and thyme camphor; and the pyrone compound is at least one selected from the group consisting of maltol, ethyl maltol, coumarin, tartaric acid, flavone and flavonoid compounds;

optionally, the carrier is preferably one or more of eugenol, tea polyphenol, thymol, maltol, ethyl maltol and alcoholic acid.

Further, the drug is at least one selected from the group consisting of taxol, rapamycin, taxol derivative and rapamycin derivative.

The present application provides a drug balloon, which is obtained by coating the aforementioned drug coating on the surface of a balloon.

The present application provides a method for preparing a drug balloon, wherein the drug balloon is the aforementioned drug balloon, and the method comprises the following steps:

dissolving the drug and the carrier into a solvent to obtain a solution; and
coating the solution onto the surface of the balloon to form a drug coating.

Further, the solvent is at least one selected from the group consisting of water, an alcohol compound, an aliphatic hydrocarbon compound, an ether compound, a halogenated hydrocarbon compound, a ketone compound, tetrahydrofuran and acetonitrile; optionally, the aliphatic hydrocarbon compound comprises n-hexane and/or n-butane; the ether compound comprises diethyl ether and/or propylene oxide; the halogenated hydrocarbon compound comprises trichloromethane; and the ketone compound comprises acetone and/or methylbutanone.

Further, a mass ratio of the drug to the solvent is 1: (0.002-50).

Further, the alcohol compound is a compound with a carbon number of 15 or less; and the alcohol compound is at least one selected from the group consisting of ethanol, methanol, isopropanol, propanol, n-butanol, isobutanol and sec-butanol.

Further, in the dissolving step, after adding the drug and the carrier into the solvent, stirring, ultrasonic vibration and standing are performed.

Further, the standing is performed for a time ranging from 0.5h to 1.5h.

Further, the method for coating comprises spray coating, dip coating or drop coating.

The present application also provides use of a drug balloon prepared by the aforementioned method for preparing a drug balloon in dilating stenosis of blood vessels, urethra or ureteral lumen and inhibiting restenosis, the blood vessels comprise coronary blood vessels, peripheral blood vessels, intracranial blood vessels, and arteriovenous fistula blood vessels, and the urethra comprises prostatic urethra, bulbous urethra, membranous urethra, and penile urethra.

The technical solution of the present application has the following advantages.

1. The drug coating provided by the present application uses phenolic compounds or pyrone compounds as new carriers, improves the efficiency of carrier selection for the drug coating and can obtain ideal needle-shaped or column-shaped crystals, remarkably improves the quality of the drug coating, and prolongs the retention time of the drug in the treatment of blood vessels or other lumen stenosis diseases, thereby enabling the drug balloon catheter to better inhibit the restenosis of blood vessels in the treatment of vascular stenosis.

2. The drug coating provided by the present application narrows the selection range of drug coating formulas, simplifies the formula selection process, and has a simple coating preparation method, which greatly improves the firmness of the drug coating.

3. The drug balloon provided by the present application is used to dilate the stenosis and inhibit restenosis of blood vessels, urethra, and ureteral lumen.

**BRIEF DESCRIPTION OF THE DRAWINGS**

In order to illustrate the technical solutions in the specific embodiments of the present application or in the prior art more clearly, the drawings need to be used in the description of the specific embodiments or the prior art will be briefly introduced below. Obviously, the drawings in the following description show some of the embodiments of the present application, and those of ordinary skill in the art may still obtain other drawings from these drawings without creative efforts.

Figure 1 is a scanning electron microscope image of the drug coating prepared in Example 1 provided by the present application; and the scale bar in the image is 5 μm; and

Figure 2 is a scanning electron microscope image of the drug coating prepared in Comparative Example 1 provided by the present application; and the scale bar in the image is 10 μm.

**DETAILED DESCRIPTION**

The following examples are provided for a better understanding of the present application, are not limited to the best embodiments, and do not constitute a limitation on the content or scope of protection of the present application. Any product identical or similar to the present application, derived by anyone under the inspiration of the present application or by combining the present application with other features of the prior art, falls within the scope of protection of the present application.

Where specific experimental steps or conditions are not indicated in the examples, the operations or conditions of conventional experimental steps described in the literatures in the present art can be followed. The reagents or instruments used without the manufacturer indicated are conventional reagent and products that are commercially available.

The balloon material used in the examples of the present application is a self-made balloon made of nylon material. The specific preparation method is as follows.

Preforming: The two ends of the balloon material tube were stretched thin, and a certain proportion of the bubble length is reserved in the middle to obtain a preformed balloon;

Mold molding: The preformed balloon was put into the balloon mold for blow molding, and then nitrogen gas was injected into the preformed balloon. The temperature of the balloon mold was controlled to be in a range from 110°C to 120°C, and the pressure of nitrogen gas in the preformed balloon is in a range from 300 psi to 400psi.

The product number of tea polyphenol used in the examples of the present application is T821916, purchased from Macklin.

The product number of maltol used in the examples of the present application is M813318, purchased from Macklin.

The product number of eugenol used in the examples of the present application is E809010, purchased from Macklin.

The product number of ethyl maltol used in the examples of the present application is E824420, purchased from Macklin.

**Example 1**

This example provides a drug coating, which comprises a drug and a carrier. The drug was taxol, the carrier was tea polyphenol, and the solvent was acetone. The mass ratio of the drug to the carrier was 25:1, and the mass ratio of the drug to the solvent was 30: 1.

This example provides a drug balloon, which was obtained by coating the balloon surface with the aforementioned drug coating. The specific preparation method comprises the following steps:

S1. the drug and the carrier were dissolved in the solvent, then stirred for 3 minutes, then ultrasonic vibration was carried out for 15 minutes at an ultrasonic frequency of 100Hz and a temperature of 30°C, and then the solution after vibration was allowed to stand for 0.5h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Example 2**

This example provides a drug coating, which comprises a drug and a carrier. The drug was taxol, the carrier was maltol, and the solvent was acetone. The mass ratio of the drug to the carrier was 20:1, and the mass ratio of the drug to the solvent was 30: 1.

This example provides a drug balloon, which was obtained by coating the balloon surface with the aforementioned drug coating. The specific preparation method comprises the following steps:

S1. the drug and the carrier were dissolved in the solvent, then stirred for 3 minutes, then ultrasonic vibration was carried out for 15 minutes at an ultrasonic frequency of 100Hz and a temperature of 30°C, and then the solution after vibration was allowed to stand for 1h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Example 3**

This example provides a drug coating, which comprises a drug and a carrier. The drug was taxol, the carrier was eugenol, and the solvent was acetonitrile. The mass ratio of the drug to the carrier was 1:10, and the mass ratio of the drug to the solvent was 500: 1.

This example provides a drug balloon, which was obtained by coating the balloon surface with the aforementioned drug coating. The specific preparation method comprises the following steps:

S1. the drug and the carrier were dissolved in the solvent, then stirred for 3 minutes, then ultrasonic vibration was carried out for 15 minutes at an ultrasonic frequency of 100Hz and a temperature of 30°C, and then the solution after vibration was allowed to stand for 1h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Example 4**

This example provides a drug coating, which comprises a drug and a carrier. The drug was taxol, the carrier was ethyl maltol, and the solvent was isopropanol. The mass ratio of the drug to the carrier was 100:1, and the mass ratio of the drug to the solvent was 1: 50.

This example provides a drug balloon, which was obtained by coating the balloon surface with the aforementioned drug coating. The specific preparation method of the drug coating comprises the following steps:

S1. the drug and the carrier were dissolved in the solvent, then stirred for 3 minutes, then ultrasonic vibration was carried out for 15 minutes at an ultrasonic frequency of 100Hz and a temperature of 30°C, and then the solution after vibration was allowed to stand for 1h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Example 5**

This example provides a drug coating, which comprises a drug and a carrier. The drug was a taxol derivative, the taxol derivative was docetaxel (specific product number was D807092, purchased from Macklin), the carrier was maltol, and the solvent was acetone. The mass ratio of the drug to the carrier was 20:1, and the mass ratio of the drug to the solvent was 30: 1.

This example provides a drug balloon, which was obtained by coating the balloon surface with the aforementioned drug coating. The specific preparation method of the drug coating comprises the following steps:

S1. the drug and the carrier were dissolved in the solvent, then stirred for 3 minutes, then ultrasonic vibration was carried out for 15 minutes at an ultrasonic frequency of 100Hz and a temperature of 30°C, and then the solution after vibration was allowed to stand for 1h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Example 6**

This example provides a drug coating, which comprises a drug and a carrier. The drug was rapamycin, the carrier was maltol, and the solvent was acetone. The mass ratio of the drug to the carrier was 20:1, and the mass ratio of the drug to the solvent was 30: 1.

This example provides a drug balloon, which was obtained by coating the balloon surface with the aforementioned drug coating. The specific preparation method of the drug coating comprises the following steps:

S1. the drug and the carrier were dissolved in the solvent, then stirred for 3 minutes, then ultrasonic vibration was carried out for 15 minutes at an ultrasonic frequency of 100Hz and a temperature of 30°C, and then the solution after vibration was allowed to stand for 1.5h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Example 7**

This example provides a drug coating, which comprises a drug and a carrier. The drug was a rapamycin derivative, the rapamycin derivative was zotarolimus (product number: Z872686, purchased from Macklin), and the carrier was maltol, and the solvent was acetone. The mass ratio of the drug to the carrier was 20:1, and the mass ratio of the drug to the solvent was 30: 1.

This example provides a drug balloon, which was obtained by coating the balloon surface with the aforementioned drug coating. The specific preparation method of the drug coating comprises the following steps:

S1. the drug and the carrier were dissolved in the solvent, then stirred for 3 minutes, then ultrasonic vibration was carried out for 15 minutes at an ultrasonic frequency of 100Hz and a temperature of 30°C, and then the solution after vibration was allowed to stand for 1.5h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Comparative Example 1**

This comparative example provides a drug coating, which comprises a drug and a carrier. The drug was taxol, the carrier was sorbitol (the product number of sorbitol was D817607, purchased from Macklin), and the solvent was acetone. The mass ratio of the drug to the carrier was 25:1, and the mass ratio of the drug to the solvent was 30:1.

This comparative example provides a drug balloon, which was obtained by coating the balloon surface with the aforementioned drug coating. The specific preparation method of the drug coating comprises the following steps:

S1. the drug and the carrier were dissolved in the solvent, then stirred for 3 minutes, then ultrasonic vibration was carried out for 15 minutes, and then the solution after vibration was allowed to stand for 0.5h; and

S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Comparative Example 2**

This comparative example provides a drug coating, which comprises a drug and a carrier. The drug was taxol, the carrier was ethyl maltol, and the solvent was propanol. The mass ratio of the drug to the carrier was 110:1, and the mass ratio of drug to the solvent was 1:50.

This comparative example provides a drug balloon, which was obtained by coating the balloon surface with the aforementioned drug coating. The specific preparation method of the drug coating comprises the following steps:

S1. the drug and the carrier were dissolved in the solvent, then stirred for 3 minutes, then ultrasonic vibration was carried out for 15 minutes, and then the solution after vibration was allowed to stand for 1h; and S2. the solution was sprayed onto the surface of the balloon through ultrasonic spraying; the spraying pressure was 0.01MPa, the spraying flow rate was 0.03mL/min, and the nominal dosage of the sprayed balloon was $1\mu g/mm^2$.

**Test Example 1**

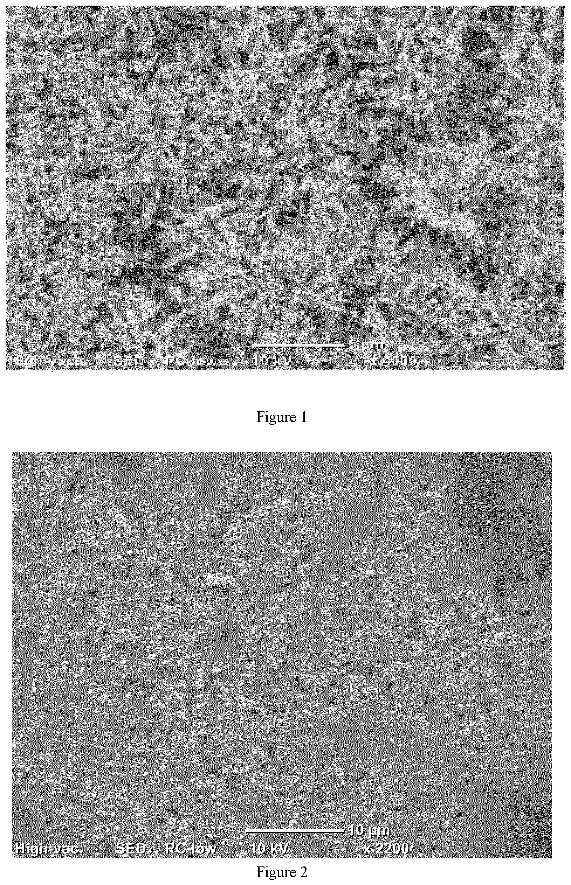

A scanning electron microscope (SEM) was used to observe the morphology of the drug coating obtained in Example 1 and Comparative Example 1 respectively. The morphology diagrams thereof were shown in Figures 1 and 2. As shown in Figures 1 and 2, the drug coating in Figure 1 formed crystal morphology of upright-growing crystals with uniform crystal sizes, mainly distributed around 5 $\mu$m, thereby allowing the drug coating to better fit the lumen wall and more crystals to be attached to the lumen wall when the drug coating was dilated in blood vessels, urethra or ureteral lumen. At the same time, the crystals had a longer metabolic time in the tissues; in Figure 2, the formed crystals were not ideal, most of them were grains with a size less than 1$\mu$m, and had a short metabolic time in the tissues.

**Test Example 2**

Balloons with a size of 2.75×12mm were selected to obtain a drug balloon according to the methods of Examples 1-7 and Comparative Examples 1-2, respectively, and the retention time of the drug balloons in the blood vessels was tested. The specific test method was as follows. The drug balloons were tested in vivo in animals. The selected animals were white pigs with a weight of about 30kg. The test blood vessel sites were the coronary artery vessels. The drug-coated balloons were delivered to the coronary artery site of the animal. After dilating for 30 seconds, the drug balloons were taken out and then the drug content of the dilated segment vascular tissue was tested on the 30th day, and the ratio of remaining drug content was calculated. The ratio of remaining drug content = tissue drug content / nominal drug content on the balloon surface × 100%. The test results were shown in Table 1.

The method for testing the drug content of the dilated segment vascular tissue was as follows. The vascular tissue was obtained, a tissue masher was used to grind the tissue homogenate at 1000r/min, and the tissue homogenate was added into 5 mL of mixed solution of methanol and acetonitrile (in which the volume ratio of methanol to acetonitrile was 1:1), then 200 $\mu$g of internal standard drug (the internal standard drug was the drug used in the corresponding examples or comparative examples) was added therein, vortex was formed for Imin, and the materials were centrifuged for 15 min at a condition of 4000 r/min to obtain a supernatant. The supernatant was diluted with asolvent of methanol and water (volume ratio of 1:1). After the supernatant was diluted to twice its volume, the tissue drug concentration data was obtained by testing using liquid chromatography-mass spectrometry.

Table 1. Intravascular test results of Examples 1-7 and Comparative Examples 1-2

| Sample group | Tissue drug content on the 30th day ($\mu$g/g) | Ratio of remaining drug content(‰) |
|---|---|---|
| Example 1 | 10.2 | 9.8 |
| Example 2 | 7.8 | 7.5 |
| Example 3 | 8.5 | 8.2 |
| Example 4 | 14.0 | 13.5 |
| Example 5 | 6.9 | 6.7 |
| Example 6 | 9.2 | 8.9 |
| Example 7 | 7.8 | 6.9 |
| Comparative Example 1 | 0.6 | 0.6 |
| Comparative Example 2 | 0.3 | 0.3 |

As shown in Table 1, for Examples 1-7, the 30th-day retention content of the drugs in coronary artery blood vessels was 10.45±3.55 $\mu$g/g, and the ratio of remaining drug content was 10.1‰±3.4‰. For Comparative Examples 1-2, the 30th-day retention content of the drugs in coronary artery blood vessels was 0.45±0.15 $\mu$g/g, and the ratio of remaining drug content was 0.45‰±0.15‰. It can be seen that the retention time of the drug in the tissue in the present application was longer than that in the comparative examples.

**Test Example 3**

Balloons with a size of 8.0×40mm were selected to obtain drug balloons according to the methods of Examples 1-7 and Comparative Examples 1-2 respectively, and the retention time of the drug balloons in the urethra was tested. The specific test method was as follows. The drug balloon was used to dilate the urethra of a dog (beagle dog of about 15kg). After 3 minutes of dilation, the drug balloon was taken out. Then, the drug content in the dilated segment urethra was tested on the 30th day, and the ratio of remaining drug content was calculated; the ratio of remaining drug content = tissue drug content/nominal drug content on the balloon surface × 100%. The test results were shown in Table 2;

The method for testing the drug content in the dilated segment urethra was as follows. The vascular tissue in the urethral tissue was obtained, a tissue masher was used to grind the tissue homogenate at 1000r/min, and the tissue homogenate was added into 5 mL of mixed solution of methanol and acetonitrile (in which the volume ratio of methanol to acetonitrile was 1:1), then 200 µg of internal standard drug (the internal standard drug was the drug used in the corresponding examples or comparative examples) was added therein, vortex was formed for 1min, and the materials were centrifuged for 15 min at a condition of 4000 r/min to obtain a supernatant. The supernatant was diluted with a solvent of methanol and water (volume ratio of 1:1). After the supernatant was diluted to twice its volume, the tissue drug concentration data was obtained by testing using liquid chromatography-mass spectrometry.

Table 2. Intraurethral test results of Examples 1-7 and Comparative Examples 1-2

| Sample group | Tissue drug content on the 30th day (µg/g) | Ratio of remaining drug content (‰) |
|---|---|---|
| Example 1 | 16.2 | 8.1 |
| Example 2 | 18.3 | 9.1 |
| Example 3 | 19.2 | 9.6 |
| Example 4 | 18.1 | 9.0 |
| Example 5 | 18.6 | 9.3 |
| Example 6 | 15.6 | 7.8 |
| Example 7 | 16.1 | 8.0 |
| Comparative Example 1 | 0.8 | 0.4 |
| Comparative Example 2 | 0.5 | 0.5 |

As shown in Table 2, for Examples 1-7, the 30th-day tissue retention amount of the drugs in the urethra was $17.4 \pm 1.8$ µg/g, and the ratio of remaining drug content was $8.6\% \pm 0.9\%$; for Comparative Examples 1-2, the 30th-day tissue retention amount of the drugs in the urethra was $0.65 \pm 0.15$µg/g, and the ratio of remaining drug content was $0.45\% \pm 0.05\%$. It can be seen that the retention time of the drug in the tissue in the present application was longer than that in the comparative examples.

**Test Example 4**

Balloons with a size of 4.0×20mm were selected to obtain drug balloons according to the methods of Examples 1-7 and Comparative Examples 1-2 respectively, and the drug release rate of the drug balloons were tested. The specific test method was as follows. The simulated blood vessels of appropriate size were immersed in the bovine serum protein PBS simulated solution, and then the above-mentioned drug balloons were inserted into the simulated blood vessels respectively and dilated to burst pressure (12 atm) and kept at this pressure for 60s, and then the pressure was relieved, and the dilated drug balloons were removed from the solution and put into a 5mL brown volumetric flask. 5mL of methanol was added therein and stood for half an hour to obtain the eluted solution of the drug. Then high performance liquid chromatography was used to measure the drug content of the eluted solution, and the residual drug rate was calculated. The test results were shown in Table 3.

The method for preparing the bovine serum protein PBS simulated solution was as follows. 10g of bovine serum protein was weighted and added into 100 mL of PBS buffer solution to prepare a 0.1g/mL of bovine serum protein solution. The method for preparing PBS buffer solution was as follows. 8g of NaCl, 0.2g of KCl, 1.44g of $Na_2HPO_4$ and 0.24g of $KH_2PO_4$ were weighed and dissolved in 800ml of distilled water, the pH value of the solution was adjusted to 7.4 with HCl, and finally distilled water was added therein to adjust the volume to 1L.

$$\text{Residual drug rate} = 1 - \text{detected drug content/nominal drug content of the drug balloon.}$$

Using the same method, the drug content was measured in the simulated blood vessel after the drug balloon was dilated, and the drug release rate was obtained. The specific calculation formulas were:

$$\text{Drug release rate} = \text{detected drug content/nominal drug content of the drug balloon}$$

$$\text{Drug loss} = 1 - \text{residual drug rate} - \text{drug release rate}$$

Table 3. Simulation test results of in vitro drug release

| Sample group | Drug release rate (%) | Residual drug rate (%) | Drug loss (%) |
|---|---|---|---|
| Example 1 | 98.1 | 1.4 | 0.5 |
| Example 2 | 97.5 | 2.0 | 0.5 |
| Example 3 | 99.2 | 0.4 | 0.4 |
| Example 4 | 98.3 | 1.3 | 0.4 |
| Example 5 | 97.8 | 1.6 | 0.6 |
| Example 6 | 99.0 | 0.3 | 0.7 |
| Example 7 | 98.7 | 1.0 | 1.3 |
| Comparative Example 1 | 78.2 | 10.4 | 11.4 |
| Comparative Example 2 | 81.1 | 9.6 | 9.3 |

As shown in Table 3, for Examples 1-7, the in vitro drug release rate was 98.35% ± 0.85%, the residual drug rate was 1.15% ± 0.85%, and the drug loss was 0.85% ± 0.45%; for Comparative Examples 1-2, the in vitro drug release rate was 79.65% ± 1.45%, the residual drug rate was 10% ± 0.4%, and the drug loss was 10.35% ± 1.05%. It can be seen that the drug release rate of the present application was higher than that of the comparative examples, and both the residual drug rate and drug loss were lower than that of the comparative examples.

Obviously, the above examples are merely examples made for clear description, rather limiting the implementations. For those of ordinary skill in the art, other different forms of variations or modifications can also be made on the basis of the above-mentioned description. All embodiments are not necessary to be and cannot be exhaustively listed herein. In addition, obvious variations or modifications derived therefrom all fall within the scope of protection of the present application.

**Claims**

1. A drug coating, comprising a drug and a carrier, wherein the carrier is a phenolic compound or a pyrone compound, and a mass ratio of the drug to the carrier is 1: (0.01-10).

2. The drug coating according to claim 1, wherein the phenolic compound is at least one selected from the group consisting of eugenol, thymol, grape polyphenol, tea polyphenol and thyme camphor; and the pyrone compound is at least one selected from the group consisting of maltol, ethyl maltol, coumarin, tartaric acid, flavone and flavonoid compounds;
optionally, the carrier is at least one selected from the group consisting of eugenol, tea polyphenol, thymol, maltol, ethyl maltol and alcoholic acid.

3. The drug coating according to claim 1 or 2, wherein the drug is at least one selected from the group consisting of taxol, rapamycin, taxol derivative and rapamycin derivative.

4. A drug balloon, wherein the drug balloon is obtained by coating the drug coating according to any one of claims 1 to 3 on the surface of a balloon.

5. A method for preparing a drug balloon, wherein the drug balloon is the drug balloon according to claim 4, and the method comprises the following steps:

dissolving the drug and the carrier into a solvent to obtain a solution; and
coating the solution onto the surface of the balloon to form a drug coating.

6. The method for preparing a drug balloon according to claim 5, wherein the solvent is at least one selected from the group consisting of water, an alcohol compound, an aliphatic hydrocarbon compound, an ether compound, a halogenated hydrocarbon compound, a ketone compound, tetrahydrofuran and acetonitrile;
optionally, the aliphatic hydrocarbon compound comprises n-hexane and/or n-butane; the ether compound comprises diethyl ether and/or propylene oxide; the halogenated hydrocarbon compound comprises trichloromethane; and the ketone compound comprises acetone and/or methylbutanone.

7. The method for preparing a drug balloon according to claim 5 or 6, wherein a mass ratio of the drug to the solvent is 1: (0.002-50).

8. The method for preparing a drug balloon according to any one of claims 5 to 7, wherein the alcohol compound is a compound with a carbon number of 15 or less;
optionally, the alcohol compound is at least one selected from the group consisting of ethanol, methanol, isopropanol, propanol, n-butanol, isobutanol and sec-butanol.

9. The method for preparing a drug balloon according to any one of claims 5 to 8, wherein in the dissolving step, after adding the drug and the carrier into the solvent, performing stirring, ultrasonic vibration and standing.

10. The method for preparing a drug balloon according to claim 9, wherein the standing is performed for a time ranging from 0.5h to 1.5h.

11. The method for preparing a drug balloon according to any one of claims 5 to 10, wherein the method for coating comprises spray coating, dip coating or drop coating.

12. Use of a drug balloon prepared by the method for preparing a drug balloon according to any one of claims 5 to 11 in dilating stenosis of blood vessels, urethra or ureteral lumen and inhibiting restenosis, the blood vessels comprise coronary blood vessels, peripheral blood vessels, intracranial blood vessels, and arteriovenous fistula blood vessels, and the urethra comprises prostatic urethra, bulbous urethra, membranous urethra, and penile urethra.

Figure 1

Figure 2

## INTERNATIONAL SEARCH REPORT

| International application No. |
| --- |
| **PCT/CN2022/082303** |

### A. CLASSIFICATION OF SUBJECT MATTER

A61L 29/08(2006.01)i; A61M 25/10(2013.01)i; A61L 31/00(2006.01)i

According to International Patent Classification (IPC) or to both national classification and IPC

### B. FIELDS SEARCHED

Minimum documentation searched (classification system followed by classification symbols)

A61L29/-; A61M25/-; A61L31/-

Documentation searched other than minimum documentation to the extent that such documents are included in the fields searched

Electronic data base consulted during the international search (name of data base and, where practicable, search terms used)

WPI, EPODOC, CNKI, CNPAT: 乐普, 药物, 涂层, 载体, 赋形剂, 成核剂, 吡喃酮, 酚, 茶多酚, 麦芽酚, 球囊, 质量比, medical, coat+, carrier, excipient, pyrone, phenol, balloon, mass, ratio

### C. DOCUMENTS CONSIDERED TO BE RELEVANT

| Category* | Citation of document, with indication, where appropriate, of the relevant passages | Relevant to claim No. |
| --- | --- | --- |
| X | CN 112535793 A (RONGCHONG (SHENZHEN) BIOMEDICAL TECHNOLOGY CO., LTD.) 23 March 2021 (2021-03-23) description, paragraphs [0035]-[0044] and [0070], and figure 1 | 1-12 |
| Y | CN 112535793 A (RONGCHONG (SHENZHEN) BIOMEDICAL TECHNOLOGY CO., LTD.) 23 March 2021 (2021-03-23) description, paragraphs [0035]-[0044] and [0070], and figure 1 | 1-12 |
| Y | CN 1042080 A (NIPPON SHINYAKU CO., LTD.) 16 May 1990 (1990-05-16) description, pages 9-10 | 1-12 |
| A | CN 102698277 A (ZHONG SHUGUANG) 03 October 2012 (2012-10-03) entire document | 1-12 |
| A | CN 108211094 A (LIFETECH SCIENTIFIC (SHENZHEN) CO., LTD.) 29 June 2018 (2018-06-29) entire document | 1-12 |
| A | CN 107376030 A (LEPU MEDICAL TECHNOLOGY (BEIJING) CO., LTD.) 24 November 2017 (2017-11-24) entire document | 1-12 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 June 2022** | **29 June 2022** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/082303** |

**C.** **DOCUMENTS CONSIDERED TO BE RELEVANT**

| Category* | Citation of document, with indication, where appropriate, of the relevant passages | Relevant to claim No. |
|---|---|---|
| A | CN 107898757 A (SHANDONG NORMAL UNIVERSITY) 13 April 2018 (2018-04-13)<br>entire document | 1-12 |
| A | CN 103372234 A (BYD CO., LTD.) 30 October 2013 (2013-10-30)<br>entire document | 1-12 |
| A | US 2008255509 A1 (LUTONIX, INC.) 16 October 2008 (2008-10-16)<br>entire document | 1-12 |

Form PCT/ISA/210 (second sheet) (January 2015)

**INTERNATIONAL SEARCH REPORT**
**Information on patent family members**

International application No.

**PCT/CN2022/082303**

| Patent document cited in search report | | | Publication date (day/month/year) | Patent family member(s) | | | Publication date (day/month/year) |
|---|---|---|---|---|---|---|---|
| CN | 112535793 | A | 23 March 2021 | CN | 112535793 | B | 08 October 2021 |
| CN | 1042080 | A | 16 May 1990 | EP | 0315079 | A1 | 10 May 1989 |
| | | | | KR | 890006225 | A | 12 June 1989 |
| | | | | DE | 3853191 | D1 | 06 April 1995 |
| | | | | JP | H02203 | A | 05 January 1990 |
| | | | | JP | H0798740 | B2 | 25 October 1995 |
| | | | | KR | 960013281 | B1 | 02 October 1996 |
| | | | | US | 5651991 | A | 29 July 1997 |
| | | | | CN | 1048182 | C | 12 January 2000 |
| CN | 102698277 | A | 03 October 2012 | CN | 106074417 | A | 09 November 2016 |
| | | | | CN | 106075457 | A | 09 November 2016 |
| | | | | CN | 102698277 | B | 03 August 2016 |
| CN | 108211094 | A | 29 June 2018 | | None | | |
| CN | 107376030 | A | 24 November 2017 | CN | 107376030 | B | 07 August 2020 |
| CN | 107898757 | A | 13 April 2018 | CN | 107898757 | B | 05 May 2020 |
| CN | 103372234 | A | 30 October 2013 | CN | 103372234 | B | 22 April 2015 |
| US | 2008255509 | A1 | 16 October 2008 | US | 2016263076 | A1 | 15 September 2016 |
| | | | | US | 2015224234 | A1 | 13 August 2015 |
| | | | | US | 2013197435 | A1 | 01 August 2013 |
| | | | | US | 8414526 | B2 | 09 April 2013 |
| | | | | US | 9033919 | B2 | 19 May 2015 |
| | | | | US | 9289537 | B2 | 22 March 2016 |
| | | | | US | 9757351 | B2 | 12 September 2017 |

Form PCT/ISA/210 (patent family annex) (January 2015)